# EUROPEAN PATENT SPECIFICATION

(11) **EP 2 531 100 B1**
(45) Date of publication and mention of the grant of the patent: **20.09.2017**
(21) Application number: 10703435.7
(22) Date of filing: 01.02.2010
(51) Int. Cl.: A61B 5/0476, A61B 5/00, G06F 19/00

(54) **PORTABLE EEG MONITOR SYSTEM WITH WIRELESS COMMUNICATION**
TRAGBARES EEG-MONITORSYSTEM MIT DRATHLOSER KOMMUNIKATION
SYSTÈME PORTABLE DE SURVEILLANCE D'ÉLECTROENCÉPHALOGRAMME (EEG) AVEC COMMUNICATION SANS FIL

(43) Date of publication of application: 12.12.2012
(73) Proprietor: T&W Engineering A/S, 3540 Lynge (DK)
(72) Inventor: KIDMOSE, Preben, DK-8320 Maarslet (DK); WESTERMANN, Søren Erik, DK-3050 Humlebak (DK)
(86) International application number: PCT/EP2010/051150
(87) International publication number: WO 2011/091856

(56) References cited:
- WO-A1-2007/004089
- WO-A1-2009/100654
- WO-A2-2007/047667
- US-A1- 2004 152 961
- US-A1- 2005 177 029
- US-A1- 2007 149 952
- US-A1- 2007 197 881
- US-A1- 2008 027 348
- US-A1- 2008 183 097
- US-A1- 2009 062 678
- US-A1- 2009 062 682
- US-A1- 2009 287 107
- US-B1- 6 354 299

## Description

The present invention relates to a system for providing an alarm of an imminent seizure of a person, where the system is based on surveillance of the electroencephalography (EEG) signal of the person. More specifically the invention relates to a system for remote surveillance of an EEG signal of a person susceptible of having a hypoglycemic seizure, where the system comprises an EEG sensor having electrodes for measuring one or more EEG signals from said person. The system further comprises a processing unit, having EEG signal processing means for analyzing the EEG signal, said signal processing means being adapted for, based on the EEG signal, identifying or predicting a hypoglycemic seizure, in said person, said processing part comprising decision means for deciding, based on said analyzed EEG signal, when an alarm or information must be provided.

For persons with diabetes accurate control of blood sugar concentration is important. The level should not be too high in order to limit the risk of long term effects of diabetes. The blood sugar level should also not be too low, since this might lead to hypoglycemia, where the person becomes absent and may become unconscious. Hypoglycemic attacks may be fatal. Often persons with diabetes have the problem that they will not feel any warning before the blood sugar concentration has fallen to a level where a hypoglycemic attack occurs. This phenomenon is known as hypoglycemic unawareness. The risk of an attack therefore often limits the possible activities of the people concerned, which furthermore decreases their quality of life. Attacks can be prevented in a simple way, e.g. by consuming appropriate food when glucose values become critically low. The number of people in the risk group is approximately 10 million.

WO-A1-2006066577 discloses an apparatus for prediction and warning of hypoglycemic attacks where an implanted unit only operates as an interface unit with a low power draw, which gathers EEG signals from one or more electrodes and transmits them wirelessly through the skin to an external unit. The external unit contains the more power demanding components including the signal processing unit and the alarm signal device. In this case the alarm may be an acoustic signal.

WO-A1-2006066577 further discloses an apparatus for prediction and warning of hypoglycemic attacks where an implanted unit contains both signal processor, alarm device, a rechargeable battery that is charged wirelessly through the skin by an external unit and a wireless communication circuit that allows the implanted unit to transfer data to the external unit or one or more substituting external units.

WO-A2-2007047667 discloses an apparatus containing a bioelectric measurement system, a remote monitoring system and mobile devices. The bioelectric measurement system is utilized in connection with a patient undergoing medical surveillance to measure bioelectric signal patterns associated with EEG and EMG readings. The remote monitoring system and the mobile devices are configured to receive transmissions of bioelectric related data from the bioelectric measurement system. If an emergency characteristic is detected, the remote monitoring system may be directed to transmit the appropriate data and/or predefined notification alarms and messages to a medically enabled mobile device.

WO-A2-2008092133 discloses a system for monitoring a subject and its susceptibility for having a seizure. The system contains an implanted assembly and an external assembly. The external assembly may be used to provide a warning instruction. The output from the external assembly may be visual, audio, tactile (e.g. vibratory), or some combination thereof. The disclosed systems can also include an alert that is configured to indicate that there is a communication error between the implanted assembly and the external assembly. The alert can be a visible alert, an audible alert, a tactile alert, or any combination thereof. The components of the external assembly may be integrated within a housing of a typical consumer electronics device such as an MP3 player or cellular phone. The radio-frequency used to transfer data between the implantable assembly and the external assembly is at a frequency typically between 13.56 MHz and 10 GHz.

WO-A2-2007150003 discloses a system for ambulatory, long term monitoring of a physiological signal from a patient. At least part of the system may be implanted within the patient. Brain activity signals are sampled from the patient with an externally powered leadless implanted device and transmitted to a handheld patient communication device for further processing. The external device will typically include a user interface. The user interface may be used to provide a warning signal when the implanted device is out of communication range.

A large part of people with diabetes are elderly persons, of which some cannot always be trusted to take the necessary actions themselves on an alarm of an imminent hypoglycemic attack. The same may be the case for children having diabetes. For persons who may need assistance in preventing an imminent hypoglycemic attack it is necessary to have a system which can also transmit an alarm to a helper, e.g. caregiver, who is not necessarily in the same room. In the case of children the helper would typically be a parent. In order to avoid errors it is essential that the handling of such a system is as simple as possible, and that it is easy to control the system. Simple handling includes that intervals between changing or recharging batteries should be as long as possible. Easy control of the system includes that it should be easy to check correct positioning of the part arranged on the skin surface of the body. Simple handling and easy control is especially important in a nursing home where one helper or caregiver may need to watch out for more than one alarm system related to different persons and at the same time be doing a number of other tasks.

The known systems for warning of an imminent hypoglycemic attack do not adequately fulfill the demands for obtaining a system which can warn a helper wirelessly with an easily handled and controlled system.

This problem has been solved by a system for remote surveillance of an EEG signal of a person susceptible of having a hypoglycemic seizure, where the system comprises an EEG sensor having electrodes for measuring one or more EEG signals from said person. The system further comprise a processing unit adapted to be removable arranged at the ear of said person, having EEG signal processing means for analyzing the EEG signal, said signal processing means being adapted for, based on the EEG signal, identifying or predicting a hypoglycemic seizure, in said person, said processing part comprising decision means for deciding, based on said analyzed EEG signal, when an alarm or information must be provided. The system also comprises alarm means for providing the person with an alarm. The system further comprise a first connection between the EEG sensor part and the processing unit, adapted for transmitting EEG signal from the EEG sensor part to the processing unit. The system further comprises an external part adapted to receive wirelessly an alarm from said processing part, said external part being adapted to forward an alarm to a helper, and the system comprises a second and wireless connection between the processing unit and the external part.

This solution has the advantage that correct positioning of the processing unit is easy to control if the housing of the control unit is arranged at the ear. It can directly be seen if it is correctly positioned. Furthermore, the part of the system arranged on the persons head can be made relatively small and of low weight.

It is also an advantage that the signal processing is performed directly in the processing unit. Thereby the EEG signal as such does not need to be transmitted to the external part. Only alarms or other specific information needs to be transmitted. This saves power for transmission.

In a preferred embodiment of the system for remote surveillance of an EEG signal the system comprises alarm means for providing the person with an alarm, such that the system is capable of giving a direct alarm to the person under surveillance for hypoglycemia. The alarm means could be a loudspeaker or a mechanical vibrator, and could be arranged in the processing unit. One advantage of both giving a direct alarm to the person and sending an alarm wirelessly to a helper, could be in situations where the person is in a period of training in order to learn to handle an alarm on his or her own. During such a period where the person may be getting used to the system, the alarm to the helper or caregiver may be an extra safety precaution to avoid hypoglycemic attacks.

Another advantage of a system capable of giving a wireless alarm to a helper as well as a direct alarm to the person in risk of hypoglycemia is that it will not be necessary to change the system as the needs for direct alarm and wireless alarm to a helper changes. Such a change could be that a child learns to handle the alarm on its own and therefore no longer needs the backup from a parent. An older person may also initially be able to handle an alarm, but may at some point in time need the safety of a helper who also gets the alarm. Logistically it will be simpler, and thereby cheaper, to manufacture and distribute one system capable of doing both, instead of two different systems.

A further advantage is that the possibility for both a direct alarm to the person and for the wireless alarm to the care giver implies that the same version or model of the EEG sensor and the processing unit can be applied both when only an alarm to the person is necessary and also when only an alarm to the care giver is necessary. Thereby the necessary number of different versions or models which need to be manufactured can be reduced.

In a preferred embodiment of the system for remote surveillance of an EEG signal the EEG sensor part is adapted for implantation on the head. This implantation may be subcutaneous or intra cranial. The advantage is that a better contact between electrodes and tissue can be obtained. A subcutaneous EEG sensor can also be implanted relatively easy. Alternatively, the EEG sensor part could be arranged in the ear canal with the electrodes in contact with the tissue in the ear canal. Thereby any kind of surgery is avoided, but the contact between the electrodes and the tissue is less stable.

In a further embodiment of the system with an implanted EEG sensor part the first connection is wireless and the EEG sensor part comprises a first coil and the processing unit comprises a second coil. The first wireless connection is based on an inductive coupling between the first and the second coils, and the first wireless connection is adapted for transferring power from the processing unit to the implantable EEG sensor part. This facilitates that the implanted EEG sensor part can be driven without a battery since it is powered from the processing unit.

In a preferred embodiment of the system the described external part is adapted for sending an alarm or information to a remote part. This has the advantage that the distance between the helper and the person in risk of a hypoglycemic seizure can be larger.

In a further embodiment of the system the remote part is a mobile phone, and said alarm or information is send over the standard mobile phone network. This has the advantage that the care giver does not need to carry around any extra equipment, and can easily survey several persons by the use of the same mobile phone.

In a further embodiment of the system the remote part is set up to send instructions to the external part or to the processing unit. This could facilitate easy tracking of the external part or to the processing unit, in case one or both of these are missing, by triggering them to make a sound. It could also trigger one or both of them to return specific information.

In a further embodiment of the system the external part is provided with at least one further function selected from the group: remote control, data logger, streaming of data to the processing unit, terminal for entering information about the handling of an alarm. If the person can apply the external part for one or more of these purposes it will increase the functionality of the system. A remote control could be applied for selecting between specific programs e.g. depending on the physical status of the person such as sleeping, awake or doing exercise. A data logger could be applied for logging larger amount of data, e.g. if it is relevant in a period to log the EEG signal, the processing unit could be set up to stream the signal to the external part which can be supplied with more data logging capacity than the more limited spacing of the processing unit would facilitate. The external part could be used for streaming data, such as music or radio, to the processing unit from where it could be played to the person through a loudspeaker forming the alarm means. The external part may be applied as a terminal, such as a keyboard, for the system to verify the mental status of the person. If an alarm has been given, the system may ask the person to respond that the necessary steps, such as eating or drinking, to avoid hypoglycemia have been taken. Further to this, the system could be set up for checking if the person has signs on hypoglycemia. This could be by asking the person to enter a code or the result of a simple calculation on the external part.

In a further embodiment of the system the second wireless connection between the processing unit and the external part is based on an inductive link. The antennae for this may be smaller compared to antennae for traditional radio communication.

In a second aspect of the invention it is also directed to a method for EEG surveillance, detecting and supplying an alarm of an imminent hypoglycemic seizure in a person.

Embodiments of the invention will now be explained in further detail with reference to the figures.
Figure 1 illustrates an embodiment where the processing unit of the system is prepared to be arranged behind the ear, and the EEG sensor part is prepared to be arranged subcutaneously in the area behind the ear.
Figure 2 illustrates schematically a system for remote surveillance of an EEG signal, based on an implanted EEG sensor part.
Figure 3 illustrates a flowchart for a method for surveillance of an EEG signal.

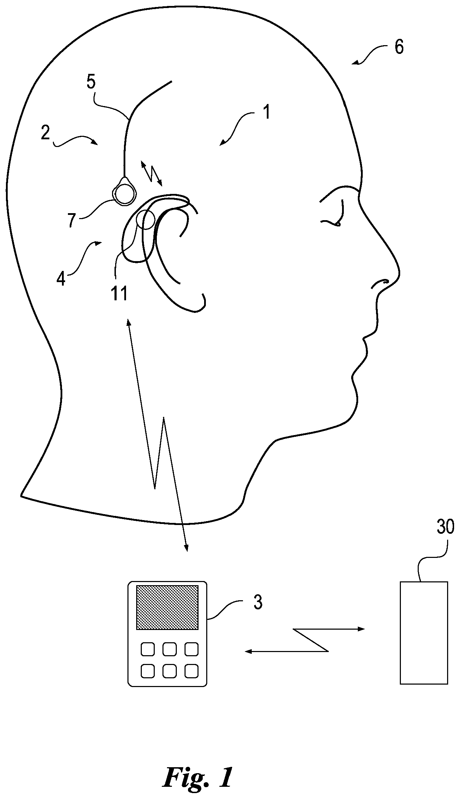

Figure 1 shows the head 6 of a person provided with a system 1 for remote surveillance of an EEG signal. The person is being monitored wearing the implantable EEG sensor 2 and the processing unit 4. The two units 2, 4 are adapted to be in wireless communication through the skin of the person. The implanted EEG sensor 2 comprises electrodes 5. The sensor will have at least two electrodes, which may be arranged as separate electrodes along the same wire 5 as illustrated in figure 1. One wire comprising all electrodes associated with respective conductors may facilitate the implantation process. The EEG sensor is provided with an electronic module 7.

The processing unit 4 is arranged at the ear of the person of whom the EEG signal is being monitored. Preferably, the processing unit 4 is arranged in a housing behind the ear as a behind the ear hearing aid. This also facilitates a position as close as possible to the implanted part, which is important for the wireless communication and power transfer through the skin. With the position behind the ear of the housing, means for fixation is also necessary. For this purpose part of a wire or sound tubing also applied for conveying the sound from a hearing aid into the ear canal may be used. This means that this wire or sound tubing should be relatively stiff, i.e. adapted for keeping the original given shape during use. Further to this purpose the wire or sound tubing can also be used for providing a sound alarm or message to the user. In the case of a wire the loudspeaker or receiver could be arranged within or close to the ear canal. In the case of a sound tubing the loudspeaker or receiver could be arranged in the housing of the processing unit.

Figure 1 further illustrates an external part 3 and a remote part 30.

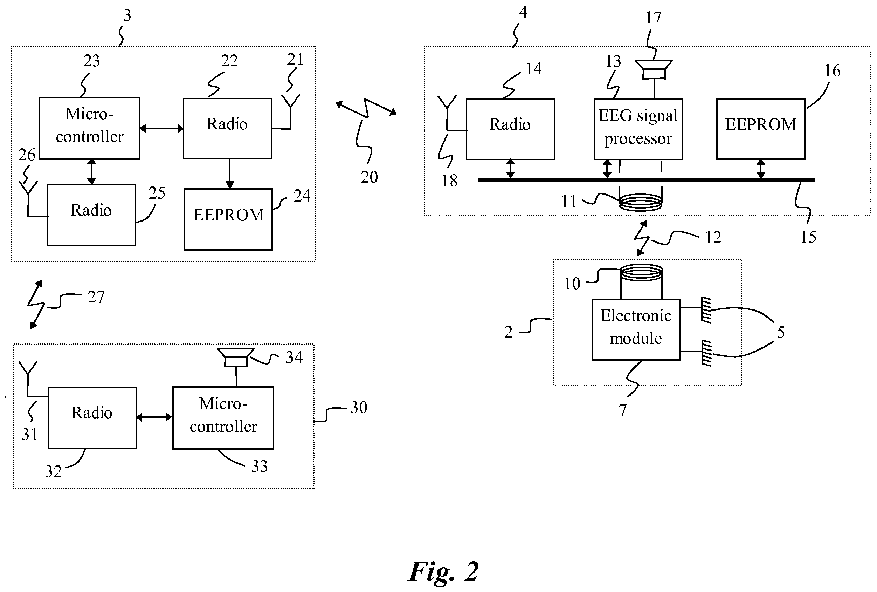

Figure 2 illustrates main components of an embodiment of a system for surveillance of an EEG signal.

The wireless connection 12 between the implantable EEG sensor 2 and the processing unit 4 is based on an inductive coupling between a coil 10 in the sensor and a coil 11 in the processing unit. This wireless connection 12 is applied to transfer power from the processing unit 4 to the implantable EEG sensor 2. Thereby, it is possible to operate the EEG sensor without a battery. The two coils 10, 11 should be closely aligned on each side of the skin barrier in order to achieve an efficient power transfer. The center axis of one coil should preferably continue through the center axis of the other coil, or the two center axes should be arranged close to each other. This close arrangement is ensured when arranging the processing unit. The EEG sensor must be placed such during implantation that it will be easy to align the two coils, i.e. the EEG sensor should be arranged in a position where the coil 10 is at a position where it will be easy to arrange the coil of the processing unit in alignment.

In case of insufficient alignment between the two coils 10, 11 for obtaining a satisfactory power and data transmission, this can be detected by the processing unit and a notification can be sent to the external device.

The wireless connection based on inductive coupling is also applied for transmission of the EEG signal from the implanted EEG sensor 2 to the processing unit 4. This may be done by varying the load on the coil 10 in the EEG sensor. The changes in load in the EEG sensor influence the power load on the coil 11 in the processing unit. In this way an EEG signal can be transmitted from the EEG sensor to the processing unit.

The inductive coupling can be based on frequencies within a wide range. It could be a frequency around 125 kHz, which is also applied in RFID systems. Much higher frequencies may also be applied. This could be a frequency in the range 0.5 - 2 MHz. But also frequencies in the range around 10 MHz can be applied. The advantage of a higher frequency is that the antennas can be made smaller. Unfortunately, higher frequency will also lead to an increase in power consumption. These tendencies apply to inductive coupling as well as to traditional radio communication. For an inductive coupling where power is to be transferred, the coils must be in the near field of each other in order to obtain an efficient power transfer. In practice this sets the limit for how small the coils can be.

As an example on antenna size for a 1 MHz wireless connection based on inductive coupling, the coil 10 for the implanted EEG sensor can be made with an outer diameter in the range 10 - 18 mm, preferably 12 - 16 mm and more preferably about 14 mm and with a height in the direction perpendicular to the plane defining the outer circumference of the circular coil of 0.5 - 1.5 mm, preferably about 1 mm. A height, or thickness, of approximately 1 mm for an implant arranged subcutaneous between the scull and the scalp should be acceptable to most people. The corresponding coil 11 in the processing unit can have an outer diameter in the range 4 - 10 mm, preferably in the range 6 - 8 mm and a height in the range 4 - 8 mm, preferably about 6 mm. Such dimensions will be suitable to fit into a housing for the processing unit equivalent to the size of a small hearing aid. Such coil sizes will be sufficient for the power transfer from the processing unit to the implanted EEG sensor, and for the signal to be transferred from the EEG sensor to the processing unit.

The processing unit 4 comprises means for transferring power through the inductive coupling via the two coils 10, 11, and means for receiving the EEG signal obtained by the EEG sensor 2. The processing unit 4 comprises a battery (not shown) for power supply to the components of the processing unit as well as power to be transferred to the EEG sensor.

The processing unit 4 also comprises an EEG signal processor 13 for analyzing the EEG signal according to predefined algorithms in order to identify an imminent seizure.

The processing unit 4 comprises a memory, such as an EEPROM 16, for logging EEG signals, specific events or alarms.

The processing unit 4 comprises an alarm device, e.g. in the form of a loudspeaker 17. The alarm device may be connected to the EEG signal processor 13, e.g. through an amplifier. In the case where the alarm device is a loudspeaker, this may be applied for replaying speech messages stored in the memory. Such messages could ask the person being monitored to perform specific acts in order to avoid a seizure. The alarm device could also be a vibrator, which in case of an alarm vibrates, e.g. against the skin to notify the person. A vibrator could also be placed subcutaneous in connection with, or as part of the implanted EEG sensor. The person would still be able to feel any vibration.

The different components of the processing unit 4 may be connected through a data bus 15.

The processing unit 4 further comprises a radio 14 with an antenna 18. This is for communication with an external part 3 through a second wireless connection 20. This radio and antenna is set up for minimum power consumption. Therefore, the radio will be adapted for short range transmission to the external part 3. The antenna must also be relatively small in order to be fit in the housing of the processing unit. Both purposes can be achieved by application of an inductive antenna, i.e. a coil. The frequency of such an inductive antenna could be around 10 MHz. The advantage of an inductive antenna is the low power consumption in the receiver and transmitter circuit. The disadvantage is the short range the signal can be transmitted since the signal intensity declines with the distance in the power of three.

As an example on antenna size for a 10 MHz wireless connection based on inductive coupling between the processing unit 4 and the external part 3, a coil antenna 18 for the processing unit can be made with an outer diameter in the range 1 - 2 mm, preferably about 1.5 mm and with a height in the direction perpendicular to the plane defining the outer circumference of the circular coil of 4 - 8 mm, preferably about 6 mm. Such dimensions make the coil feasible for arrangement in the housing for the processing unit. The corresponding coil antenna 21 in the external part can have an outer diameter in the range 3 - 8 mm, preferably in the range 5 - 6 mm and a height in the range 20 - 50 mm, preferably in the range 30 - 40 mm.

Therefore the external part 3 to which the processing unit 4 transmits should be arranged in relatively close range of the person equipped with the EEG sensor 2 and the processing unit 4. This means that the external part should be in the same room, or maybe in the person's pocket, e.g. preferably within a range of 4 m, more preferably within 3 m, and even more preferably within 2 m.

The communication in the second wireless connection 20 between the processing unit and the external part could also be performed by a standard radio. While the communication in the first wireless connection between the implanted EEG sensor and the processing unit is more or less continuous, the communication in the second wireless connection between the processing unit and the external part is more sparse, since the signal processing of the EEG signal is performed in the processing unit, and the processing unit is only sending to the external part at specific occasions, such as in the case of a detected imminent seizure or in the case of specific maintenance related information such as low charge on battery or brief confirmation messages that the system is functioning properly. This means that only a small bandwidth is necessary for the second wireless connection due to the relatively small amount of data to be transferred. Thereby this connection will be less sensitive to noise from the surroundings.

The external part 3 comprises a short range radio 22 for communication with the processing unit 4 through an antenna 21. The external part 3 further comprises a longer range radio 25 for communication with a remote part 30. The external part 3 comprises a memory 24 to which data from the processing unit 4 can be transferred. The memory capacity in the external part 3 could be considerably larger than the memory capacity in the processing unit. The external part 3 further comprises a microcontroller 23 for handling the communication with the processing unit 4 as well as with the remote part 30. Since the external part 3 does not have to be carried directly on the body, it can be heavier and larger than the processing unit 4, and can, therefore, comprise a battery (not shown) which can have a considerably larger capacity than the battery of the processing unit 4. Thereby, the radio communication with the remote unit can be of a more power demanding type, and thus provide communication over a longer distance.

The housing of the processing unit could comprise transducers for other measurements of the actual conditions of the person being monitored. This could be temperature, skin humidity, pulse etc.

The external part 3 may also comprise other features in addition to functioning as a relay device between the processing unit 4 and a remote part 30. Such other features could be to function as a remote control in cases where a remote control is necessary. This could be in versions where different programs are possible, or for the person to respond to an alarm. Another feature could be a further alarm device, preferably of an acoustic type, for warning people close to the person with the EEG surveillance.

The remote part 30 must be in communication with the external part over a longer distance, at least to a different room in a building. This means that the communication link 27 between the radio 32 and antenna 31 in the remote part 30 and the radio 25 and the antenna 26 in the external part should be through a standard radio connection and not an inductive type. Any radio communication for a range of e.g. 10 meter or more could in principle be used for the purpose.

One possibility would be to apply the mobile telephone network for the communication link 27. By doing this the remote part 30 could be a standard mobile telephone e.g. supplied with software for giving a warning in a predefined form. Preferably, the remote part 30 has software and/or a microcontroller 33 adapted for providing information on how to handle the alarm. The illustrated remote part 30 in figure 2 has alarm means in form of a loudspeaker 34.

Data other than alarms may also be transmitted to the remote part. This could be data or information on incidences where an alarm has almost been triggered but was prevented. Prevention of an alarm could take place if the person under EEG surveillance was eating or drinking, thereby increasing the blood sugar concentration, just before the alarm would have been triggered.

It would also be relevant to transmit information on the operational condition of the EEG sensor, processing unit or external part to the care giver. This could be low battery charge. Also information on loss of contact between the processing unit and the EEG sensor is important to transmit to the remote part. Such loss of contact may be due to removal of the processing unit, or that its position has been changed such that the coils are no longer aligned. Also data indicating the physical status of the person, e.g. sleeping or awake, could be transferred. Obviously, all types of data transferred should only happen with the consent of the person.

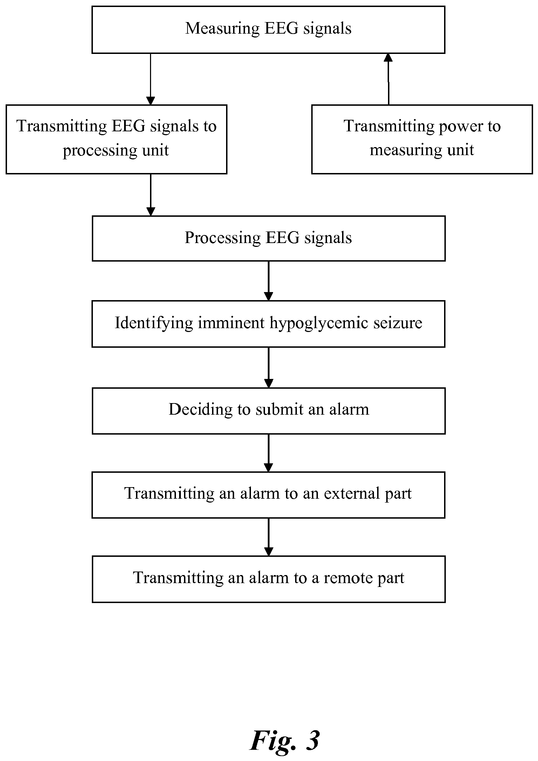

Figure 3 is a flow chart of the steps in a method for wireless surveillance for detecting and supplying an alarm concerning an imminent hypoglycemic seizure. This method is based preferably based on the embodiment with an implanted EEG sensor. The first step in the method is continuously measuring EEG signals of a person susceptible of having a hypoglycemic seizure. The EEG signals are measured by the EEG sensor 2, which is preferably implanted subcutaneous on the head of the person, preferably behind the ear. The EEG sensor is provided with electrodes 5 for measuring one or more EEG signals, and comprises a coil for wireless communication. The measured EEG signal is transmitted from the EEG sensor to the processing unit through the wireless connection, based on inductive coupling, between the first coil 10 of the EEG sensor and the second coil 11 of the processing unit 4. Furthermore, power is transmitted from the processing unit to the implantable EEG sensor, through the same inductive coupling. In a next step the EEG signal is being processed in the processing unit having EEG signal processing means for analyzing the EEG signal. Identification or prediction of an imminent hypoglycemic seizure is performed in the signal processing means, based on the EEG signal. Based on this identification or prediction a decision is made on whether or not an alarm, or other information, must be provided to the helper or the person being monitored. In a further step the processing unit transmits an alarm to an external part through a second wireless connection established between the processing unit and the external part. The external part may provide an alarm to a care giver. In a preferred embodiment the external part transmits the alarm to a remote part which provides the alarm to the care giver.

## Claims

1. A system (1) for remote surveillance of an EEG signal of a person having diabetes, said system comprising
- an EEG sensor part (2) having electrodes (5) for measuring one or more EEG signals from said person, said EEG sensor part (2) is provided with an electronic module (7),
- a processing unit (4) adapted to be removable arranged at the ear of said person, having EEG signal processing means for analyzing the EEG signal, said signal processing means being adapted for, based on the EEG signal, identifying or predicting a hypoglycemic seizure in said person, said processing part (4) comprising decision means for deciding, based on said analyzed EEG signal, when an alarm or information must be provided,
- a first connection (12) between the EEG sensor part (2) and the processing unit (4), adapted for transmitting an EEG signal from the EEG sensor part to the processing unit,
- an external part (3) adapted to receive wirelessly an alarm from said processing part (4), said external part (3) being adapted to forward an alarm to a care giver, and
- a second connection (20) between the processing unit (4) and the external part (3), said second connection (20) being wireless,
**characterized in that** said EEG sensor part being adapted for implantation in the head (6), preferably subcutaneous implantation, and **in that** said first connection (12) is wireless and said EEG sensor part (2) comprises a first coil (10) and said processing unit (4) comprises a second coil (11), wherein said first wireless connection (12) being based on an inductive coupling between said first and said second coils (10, 11) and being adapted for transferring power from the processing unit (4) to the implantable EEG sensor part (2).

2. A system according to claim 1, comprising alarm means (17) for providing said person with an alarm.

3. A system according to claim 1 or 2, wherein said external part (3) is adapted for sending an alarm or information to a remote part (30).

4. A system according to claim 3, wherein said remote part (30) is a mobile phone, and said alarm or information is sent over the standard mobile phone network.

5. A system according to claim 3 or 4, wherein said remote part (30) is set up to send instructions to said external part (3) or to said processing unit (4).

6. A system according to any one of the previous claims, wherein said external part (3) is provided with at least one further function selected from the group: remote control, data logger, streaming of data to the processing unit, terminal for entering information about the handling of an alarm.

7. A system according to any one of the previous claims, wherein said second wireless connection (20) between the processing unit and the external part is based on an inductive link.

8. A method for EEG surveillance, for providing an alarm in the event of a risk of an imminent hypoglycemic seizure in a person, said method comprising the steps of
- measuring EEG signals of said person by an EEG sensor part (2), said EEG sensor having electrodes (5) for measuring one or more EEG signals, and said EEG sensor (2) is provided with an electronic module (7),
- transmitting the measured EEG signal from the EEG sensor part to a processing unit (4) through a first connection (12) between the EEG sensor part (2) and the processing unit (4), said processing unit (4) being adapted to be removable arranged at the ear of said person,
- processing the EEG signal in said processing unit (4) having EEG signal processing means for analyzing the EEG signal, said signal processing means being adapted for, based on the EEG signal, identifying or predicting a hypoglycemic seizure in said person, said processing unit comprising decision means for deciding, based on said analyzed EEG signal, when an alarm must be provided, and
- transmitting an alarm from said processing unit (4) to an external part (3) through a second connection (20) established wirelessly between the processing unit and the external part,
**characterized in that** said first connection (12) being wireless and said EEG sensor part (2) has been implanted subcutaneous on the head (6) of the person, and **in that** said first connection (12) is wireless and said EEG sensor (2) part comprises a first coil (10) and said processing unit (4) comprises a second coil (11), wherein said first wireless connection (12) being based on an inductive coupling between said first and said second coils and is transferring power from the processing unit to the implantable EEG sensor part.

9. A method according to claim 8, further comprising the step of the external part (3) transmitting an alarm to a remote part (30).

## Patentansprüche

1. System (1) für die Fernüberwachung eines EEG-Signals einer Person, die Diabetes hat, wobei das System Folgendes umfasst
- ein EEG-Sensorteil (2), das Elektroden (5) zum Messen eines oder mehrerer EEG-Signale von der Person aufweist, wobei das EEG-Sensorteil (2) mit einem elektronischen Modul (7) ausgerüstet ist,
- eine Verarbeitungseinheit (4), die angepasst ist, am Ohr der Person entfernbar angeordnet zu sein, und die EEG-Signalverarbeitungsmittel zum Analysieren des EEG-Signals aufweist, wobei die Signalverarbeitungsmittel angepasst sind, auf Basis des EEG-Signals einen hypoglykämischen Anfall bei der Person zu identifizieren oder vorauszusagen, wobei das Verarbeitungsteil (4) Entscheidungsmittel umfasst, um auf Basis des analysierten EEG-Signals zu entscheiden, wann ein Alarm oder Informationen bereitgestellt werden müssen,
- eine erste Verbindung (12) zwischen dem EEG-Sensorteil (2) und der Verarbeitungseinheit (4), die zum Übertragen eines EEG-Signals vom EEG-Sensorteil zur Verarbeitungseinheit angepasst ist,
- ein externes Teil (3), das angepasst ist, vom Verarbeitungsteil (4) drahtlos einen Alarm zu empfangen, wobei das externe Teil (3) angepasst ist, einen Alarm zu einem Pfleger weiterzuleiten, und
- eine zweite Verbindung (20) zwischen der Verarbeitungseinheit (4) und dem externen Teil (3), wobei die zweite Verbindung (20) drahtlos ist,
**dadurch gekennzeichnet, dass** das EEG-Sensorteil für eine Implantation in den Kopf (6) angepasst ist, vorzugsweise subkutane Implantation, und dadurch, dass die erste Verbindung (12) drahtlos ist und das EEG-Sensorteil (2) eine erste Spule (10) und die Verarbeitungseinheit (4) eine zweite Spule (11) umfasst, wobei die erste drahtlose Verbindung (12) auf einer induktiven Kopplung zwischen der ersten und der zweiten Spule (10, 11) basiert und zum Transferieren von Strom von der Verarbeitungseinheit (4) zum implantierbaren EEG-Sensorteil (2) angepasst ist.

2. System nach Anspruch 1, das Alarmmittel (17) umfasst, um der Person einen Alarm bereitzustellen.

3. System nach Anspruch 1 oder 2, wobei das externe Teil (3) zum Senden eines Alarms oder von Informationen zu einem fernen Teil (30) angepasst ist.

4. System nach Anspruch 3, wobei das ferne Teil (30) ein Mobiltelefon ist und der Alarm oder die Informationen über das Mobilfunkstandardnetzwerk gesendet werden.

5. System nach Anspruch 3 oder 4, wobei das ferne Teil (30) eingerichtet ist, Anweisungen zum externen Teil (3) oder zur Verarbeitungseinheit (4) zu senden.

6. System nach einem der vorhergehenden Ansprüche, wobei das externe Teil (3) mit mindestens einer weiteren Funktion ausgerüstet ist, die ausgewählt wird aus der Gruppe, bestehend aus: Fernsteuerung, Datenlogger, Streaming von Daten zur Verarbeitungseinheit, Endgerät zum Eingeben von Informationen zur Handhabung eines Alarms.

7. System nach einem der vorhergehenden Ansprüche, wobei die zweite drahtlose Verbindung (20) zwischen der Verarbeitungseinheit und dem externen Teil auf einem induktiven Link basiert.

8. Verfahren für EEG-Überwachung zum Bereitstellen eines Alarms im Falle eines Risikos eines bevorstehenden hypoglykämischen Anfalls bei einer Person, wobei das Verfahren die folgenden Schritte umfasst
- Messen von EEG-Signalen der Person durch ein EEG-Sensorteil (2), wobei der EEG-Sensor Elektroden (5) zum Messen eines oder mehrerer EEG-Signale aufweist und der EEG-Sensor (2) mit einem elektronischen Modul (7) ausgerüstet ist,
- Übertragen des gemessenen EEG-Signals über eine erste Verbindung (12) zwischen dem EEG-Sensorteil (2) und einer Verarbeitungseinheit (4) vom EEG-Sensorteil zur Verarbeitungseinheit (4), wobei die Verarbeitungseinheit (4) angepasst ist, am Ohr der Person entfernbar angeordnet zu sein,
- Verarbeiten des EEG-Signals in der Verarbeitungseinheit (4), die EEG-Signalverarbeitungsmittel zum Analysieren des EEG-Signals aufweist, wobei die Signalverarbeitungsmittel angepasst sind, auf Basis des EEG-Signals einen hypoglykämischen Anfall bei der Person zu identifizieren oder vorauszusagen, wobei die Verarbeitungseinheit Entscheidungsmittel umfasst, um auf Basis des analysierten EEG-Signals zu entscheiden, wann ein Alarm oder Informationen bereitgestellt werden müssen, und
- Übertragen eines Alarms über eine zweite Verbindung (20), die drahtlos zwischen der Verarbeitungseinheit (4) und einem externen Teil (3) hergestellt ist, von der Verarbeitungseinheit zum externen Teil,
**dadurch gekennzeichnet, dass** die erste Verbindung (12) drahtlos ist und das EEG-Sensorteil (2) subkutan in den Kopf (6) der Person implantiert wurde, und dadurch, dass die erste Verbindung (12) drahtlos ist und das EEG-Sensorteil (2) eine erste Spule (10) und die Verarbeitungseinheit (4) eine zweite Spule (11) umfasst, wobei die erste drahtlose Verbindung (12) auf einer induktiven Kopplung zwischen der ersten und der zweiten Spule basiert und Strom von der Verarbeitungseinheit zum implantierbaren EEG-Sensorteil transferiert.

9. Verfahren nach Anspruch 8, das ferner den Schritt des Übertragens eines Alarms durch das externe Teil (3) zu einem fernen Teil (30) umfasst.

## Revendications

1. Système (1) pour la surveillance à distance d'un signal EEG d'une personne ayant du diabète, ledit système comprenant
- une partie capteur d'EEG (2) ayant des électrodes (5) pour mesurer un ou plusieurs signaux EEG provenant de ladite personne, ladite partie capteur d'EEG (2) est munie d'un module électronique (7),
- une unité de traitement (4) conçue pour être agencée de façon amovible au niveau de l'oreille de ladite personne, ayant un moyen de traitement de signal EEG pour analyser le signal EEG, ledit moyen de traitement de signal étant conçu pour, sur la base du signal EEG, identifier ou prévoir une crise hypoglycémique chez ladite personne, ladite partie de traitement (4) comprenant un moyen de décision pour décider, sur la base dudit signal EEG analysé, quand une alarme ou une information doit être fournie,
- une première connexion (12) entre la partie capteur d'EEG (2) et l'unité de traitement (4), conçue pour émettre un signal EEG de la partie capteur d'EEG à l'unité de traitement,
- une partie externe (3) conçue pour recevoir sans fil une alarme en provenance de ladite partie de traitement (4), ladite partie externe (3) étant conçue pour faire suivre une alarme vers un aide-soignant, et
- une seconde connexion (20) entre l'unité de traitement (4) et la partie externe (3), ladite seconde connexion (20) étant sans fil,
**caractérisé en ce que** ladite partie capteur d'EEG est conçue pour implantation dans la tête (6), de préférence une implantation sous-cutanée, et **en ce que** ladite première connexion (12) est sans fil et ladite partie capteur d'EEG (2) comprend une première bobine (10) et ladite unité de traitement (4) comprend une seconde bobine (11), dans lequel ladite première connexion sans fil (12) est basée sur un couplage inductif entre ladite première et ladite seconde bobine (10, 11) et est conçue pour transférer la puissance de l'unité de traitement (4) à la partie capteur d'EEG (2) pouvant être implantée.

2. Système selon la revendication 1, comprenant un moyen d'alarme (17) pour munir ladite personne d'une alarme.

3. Système selon la revendication 1 ou 2, dans lequel ladite partie externe (3) est conçue pour envoyer une alarme ou une information à une partie distante (30).

4. Système selon la revendication 3, dans lequel ladite partie distante (30) est un téléphone portable, et ladite alarme ou information est envoyée sur le réseau de téléphone portable standard.

5. Système selon la revendication 3 ou 4, dans lequel ladite partie distante (30) est paramétrée pour envoyer des instructions à ladite partie externe (3) ou à ladite unité de traitement (4).

6. Système selon l'une quelconque des revendications précédentes, dans lequel ladite partie externe (3) est munie d'au moins une fonction supplémentaire sélectionnée à partir du groupe : d'une télécommande, d'un enregistreur chronologique automatique de données, de la lecture en continu de données vers l'unité de traitement, d'un terminal pour entrer des informations concernant la manipulation d'une alarme.

7. Système selon l'une quelconque des revendications précédentes, dans lequel ladite seconde connexion sans fil (20) entre l'unité de traitement et la partie externe est basée sur une liaison inductive.

8. Procédé pour la surveillance d'EEG, pour fournir une alarme en cas d'un risque d'une crise hypoglycémique imminente chez une personne, ledit procédé comprenant les étapes de
- mesure des signaux EEG de ladite personne par une partie capteur d'EEG (2), ledit capteur d'EEG ayant des électrodes (5) pour mesurer un ou plusieurs signaux EEG, et ledit capteur d'EEG (2) est muni d'un module électronique (7),
- émission du signal EEG mesuré depuis la partie capteur d'EEG vers une unité de traitement (4) par l'intermédiaire d'une première connexion (12) entre la partie capteur d'EEG (2) et l'unité de traitement (4), ladite unité de traitement (4) étant conçue pour être agencée de façon amovible au niveau de l'oreille de ladite personne,
- traitement du signal EEG dans ladite unité de traitement (4) ayant un moyen de traitement de signal EEG pour analyser le signal EEG, ledit moyen de traitement de signal étant conçu pour, sur la base du signal EEG, identifier ou prévoir une crise hypoglycémique chez ladite personne, ladite unité de traitement comprenant un moyen de décision pour décider, sur la base dudit signal EEG analysé, quand une alarme doit être fournie, et
- émission d'une alarme à partir de ladite unité de traitement (4) à une partie externe (3) par l'intermédiaire d'une seconde connexion (20) établie sans fil entre l'unité de traitement et la partie externe,
**caractérisé en ce que** ladite première connexion (12) est sans fil et ladite partie capteur d'EEG (2) a été implantée de manière sous-cutanée sur la tête (6) de la personne, et **en ce que** ladite première connexion (12) est sans fil et ladite partie capteur d'EEG (2) comprend une première bobine (10) et ladite unité de traitement (4) comprend une seconde bobine (11), dans lequel ladite première connexion sans fil (12) est basée sur un couplage inductif entre ladite première et ladite seconde bobine et transfère de la puissance depuis l'unité de traitement vers la partie capteur d'EEG pouvant être implantée.

9. Procédé selon la revendication 8, comprenant en outre l'étape consistant à ce que la partie externe (3) émet une alarme vers une partie distante (30).
